# EUROPEAN PATENT SPECIFICATION

(11) **EP 3 989 919 B1**
(45) Date of publication and mention of the grant of the patent: **03.12.2025**
(21) Application number: 20831317.1
(22) Date of filing: 24.06.2020
(51) Int. Cl.: A61K 8/34, A61Q 13/00, A61Q 15/00, G01N 33/50, G01N 33/74

(54) **OPTIMIZATION OF SCENT OR FLAVOR COMPOSITIONS**
OPTIMIERUNG VON DUFT- ODER AROMAZUSAMMENSETZUNGEN
OPTIMISATION DE COMPOSITIONS DE PARFUM OU D'ARÔME

(30) Priority: 25.06.2019 US 201962866203 P
(43) Date of publication of application: 04.05.2022
(73) Proprietor: Suntory Holdings Limited, Osaka-shi, Osaka 530-8203 (JP)
(72) Inventor: SILVERMAN, Joshua, Los Altos Hills, CA 94022 (US); MONCADA, Morgan, Z., Belmont, CA 94002 (US); HARRIES, William, E., C., Boulder Creek, CA 95006 (US)
(74) Representative: Vossius & Partner Patentanwälte Rechtsanwälte mbB
(86) International application number: PCT/US2020/039352
(87) International publication number: WO 2020/263973

(56) References cited:
- US-A1- 2013 324 431
- US-A1- 2015 177 202
- US-A1- 2015 260 707
- US-A1- 2016 287 161
- US-A1- 2018 110 457
- US-A1- 2019 056 382
- DOTY RICHARD L. ET AL: "The influences of age on olfaction: a review", FRONTIERS IN PSYCHOLOGY, vol. 5, 7 February 2014 (2014-02-07), XP93082799, Retrieved from the Internet <URL:https://www.ncbi.nlm.nih.gov/pmc/articles/PMC3916729/pdf/fpsyg-05-00020.pdf> DOI: 10.3389/fpsyg.2014.00020
- DOTY RICHARD L. ET AL: "The influences of age on olfaction: a review", vol. 5, 7 February 2014 (2014-02-07), XP093082799, Retrieved from the Internet <URL:https://www.ncbi.nlm.nih.gov/pmc/articles/PMC3916729/pdf/fpsyg-05-00020.pdf> DOI: 10.3389/fpsyg.2014.00020
- BACCARANI ALESSIA ET AL: "The influence of stimulus concentration and odor intensity on relaxing and stimulating perceived properties of odors", FOOD QUALITY AND PREFERENCE, ELSEVIER, AMSTERDAM, NL, vol. 87, 15 July 2020 (2020-07-15), XP086276114, ISSN: 0950-3293, [retrieved on 20200715], DOI: 10.1016/J.FOODQUAL.2020.104030
- ROSPARS JEAN-PIERRE, LÁNSKÝ PETR, DUCHAMP-VIRET PATRICIA, DUCHAMP ANDRÉ: "Spiking frequency versus odorant concentration in olfactory receptor neurons", BIOSYSTEMS, vol. 58, no. 1-3, 1 December 2000 (2000-12-01), pages 133 - 141, XP055780229, ISSN: 0303-2647, DOI: 10.1016/S0303-2647(00)00116-7

## Description

### FIELD

This application relates to methods as defined in the appended claims for identifying olfactory receptors capable of binding odorant compositions that are differentially perceived by at least two age groups. The identified olfactory receptors may be used to generate an age-related odor or flavor profile that determines an effective concentration range of the odorant compositions for receptor stimulation. In turn, the odor or flavor profiles may be used to either optimize flavors and odors to appeal to certain age groups, or to discourage use by certain age groups. In some instances, odorant compositions may be screened to determine whether they are differentially perceived by age groups.

### BACKGROUND

Odor molecules belong to a variety of chemical classes including, for example, alcohols, aldehydes, ketones and carboxylic acids, sulfur-containing compounds, and essential oils. Odorant compositions generally include odorant molecules that bind one or more corresponding olfactory receptors that are associated with a particular perceived odor or taste. Some scientific studies have shown that certain olfactory perceptions diminish as humans age, such that higher concentrations of an odorant molecule may be needed in order for an adult to perceive the same level of intensity of the odorant molecule compared to that perceived by a child or an adolescent. This declining olfaction capability may be similar to the phenomenon of adults losing sensitivity to certain auditory frequencies as they age. The difference in perception may be due to age-related downregulation of the olfactory receptor, or other interference along the perception neurological pathway. Downregulation of receptors may also appear with neurological disorders such as Parkinson's disease and Alzheimer's disease, or other physiological states. Moreover, US 2015/260707 relates to a method for evaluating the scent performence of perfume mixtures.

The cannabis industry has experienced a significant increase in new product development including beverages and edible cannabinoid variants. Similarly, the tobacco industry has experienced an increase in development of new vape emission (e-cigarette) products to obviate the health consequences of traditional lit-end cigarettes. These new products, however, have met with considerable backlash from critics claiming that the new products are designed to appeal to children and adolescents. Thus far, this a complicated assertion to prove or disprove since age-related components of taste and smell perception, as well as the science underlying these phenomena, are poorly understood. Most studies have focused on consumer surveys or focus groups, which are unreliable since they are based on subjective interpretations.

Accordingly, it would be useful to design and optimize flavors and odors to appeal to certain age groups, or conversely, to create flavors and odors that are unappealing, and which discourage use by certain age groups. These designed flavors and odors may be included in various consumer products, for example, age-restricted products such as alcohol, tobacco, or cannabis. The designed odors may also be used in certain environments to attract or increase their appeal to certain age groups.

### SUMMARY

Described herein are methods as defined in the appended claims for identifying olfactory receptors capable of binding odorant compositions that are differentially perceived by at least two age groups. The identified olfactory receptors may be used to generate an age-related odor or flavor profile that determines an effective concentration range of the odorant compositions for stimulation of the receptors. In turn, the odor or flavor profiles may be used to either optimize flavors and odors to appeal to certain age groups, or to discourage use by certain age groups. The odorant compositions used in claim 1 (i.e. the method of the invention in its general sense) are differentially perceived by age groups.

The odorant compositions may be added to consumer products or to various environments at concentrations that elicit a desired response in certain age groups. For example, at certain concentrations the odorant composition may function to deter use of an age-restricted product by children and adolescents. Odorant compositions including menthol at high concentrations may be useful deterrents for this age group.

In general, the methods described herein may include one or more of the following steps: assaying olfactory receptors with an odorant composition to determine the effective concentration range for stimulation of the receptors, identifying an odorant composition and/or corresponding olfactory receptors that result in differential perception by one age group over another, adding the odorant composition to a consumer product at an appropriate concentration such that it causes a preferential response in one age group over another, re-measuring the stimulation of the identified receptors for the target response in the context of the final consumer product to confirm the desired level of activity, and adjusting the concentration of the odorant composition based on interference or synergy with the consumer product mixture.

A method for determining the concentration of an odorant composition for use with a product may include: 1) identifying one or more olfactory receptors that bind the odorant composition, where the odorant composition is differentially perceived by at least two age groups , and wherein identifying is performed by: a) exposing the one or more olfactory receptors to the odorant composition at a plurality of odorant concentrations, the one or more olfactory receptors being coupled to a reporter that produces a signal upon binding of the odorant composition to the one or more olfactory receptors; and b) detecting binding of the one or more olfactory receptors to the odorant composition at each odorant concentration by measuring the produced signal; 2) creating an age-related odor or flavor profile by determining the concentration range of the odorant composition resulting in a 10% to 90% maximal binding response to the one or more odorant receptors (i.e. EC₁₀ to EC₉₀ concentration range); and 3) designating for use the odorant composition in the product at the concentration that produces the desired effect in one of the at least two age groups.

The odorant composition may be included in a product or environment at the concentration that produces the desired effect in a certain age group. For example, an odorant composition designed for adults may be included in a product at an EC₁₀ to greater than EC₉₀ concentration to produce a negative effect that deters use of the product in children and adolescents. Exemplary odorant compositions that are generally differentially perceived by age groups include menthol, mint, banana, onion, and rose.

The one or more olfactory receptors may be part of a live cell assay that uses yeast or mammalian cells. Alternatively, the olfactory receptors may be provided in an assay that uses membrane fractions of a host cell. In some instances, the one or more olfactory receptors may be provided in liposomes.

Methods for deterring use of an age-restricted product by children or adolescents are also described herein. These methods generally include detecting binding of an odorant composition at a plurality of concentrations by one or more olfactory receptors as described above, and specifically designating use of menthol in the odorant composition at a concentration ranging from the EC₈₀ to greater than EC₉₀ concentration for its target olfactory receptor(s). Exemplary age-restricted products include alcohol, cannabis, nicotine, and tobacco.

### DETAILED DESCRIPTION

Described herein are methods as defined in the appended claims for identifying olfactory receptors capable of binding odorant compositions that are differentially perceived by at least two age groups. The identified olfactory receptors may be used to determine an effective concentration range of the odorant compositions for receptor stimulation that generate specific age-related odor or flavor profiles. In turn, the odor or flavor profiles may be used to either optimize flavors and odors to appeal to certain age groups, or to discourage use by certain age groups. In some instances, odorant compositions may be screened to determine whether they are differentially perceived by age groups.

With respect to age groups, the difference in perception is generally understood to be between persons in two different age groups, for example, between an older person and a younger person. The older person may be about 30 years of age, and the younger person may be about 10 years of age. In some instances, the older person may be about 40 years of age, and the younger person may be about 20 years of age. In other instances, the older person may be about 50 years of age, and the younger person about 30 years of age. Age groups may also refer to adults and children. Adults are generally persons that are above the legal age for consumption of age-restricted products including alcohol, tobacco, e-cigarettes, and cannabis. In the United States, this generally includes persons 18 years of age and older for tobacco and e-cigarette use, and persons 21 years of age and older for alcohol and cannabis use. In other areas of the world, the legal age for consumption of age-restricted products may vary between 15 and 25 years of age and older. A subset of the adult age group is the senior age group, which includes persons about 65 years of age and older. Children may generally be persons less than the minimum age for consumption of age-restricted products, which is 18 years of age in the United States. A subset of the child age group is the adolescent age group, which generally includes persons between the ages of about 10 and 17.

The differentially perceived odorant compositions may be included in age-restricted products at the concentration that optimizes a desired response, as stated above. This may be useful for the alcohol, tobacco, e-cigarette, and cannabis industries to better target adult consumers and allow for assessment of age-differentiating flavor profiles without direct testing in children. Age-restricted products are generally products that are legally limited for use by certain age groups.

In some variations, the differentially perceived odorant compositions may be used in certain environments to attract or increase their appeal to certain age groups. For example, these compositions may be used in the home to provide desired responses for either adults or children in the home.

The odorant compositions described herein may be compositions including one or more odorant molecules that can be detected by at least one olfactory receptor, and which are associated with a perceived odor or flavor. As used herein, the terms "odorant molecule" and "odorant" are used interchangeably.

The olfactory receptors ("ORs") described herein may be human olfactory receptors, olfactory receptors found in non-human animals, trace amine associated receptors, vomeronasal receptors, formyl peptide receptors, membrane guanylyl cyclase, subtype GC-D receptors, and G-protein coupled taste receptors. Olfactory receptors may also be hybrid receptors made from olfactory receptors, trace amine associated receptors, vomeronasal receptors, formyl peptide receptors, membrane guanylyl cyclase, subtype GC-D receptors, and G-protein coupled taste receptors. Additionally, olfactory receptors may be hybrid or chimeric receptors made from olfactory receptors and signaling receptor proteins native to yeast or mammalian cell types, or codon optimized olfactory receptors or those engineered via directed evolution. Furthermore, olfactory receptors may refer to ectopic olfactory receptors and olfactory receptors in the nasal cavity.

In some variations, the methods described herein include one or more of the following: assaying olfactory receptors with an odorant composition to determine the effective concentration range for stimulation of the receptors, identifying an odorant composition and/or corresponding olfactory receptors that result in differential perception by one age group over another, adding the odorant composition to a consumer product at an appropriate concentration such that it causes a preferential response in one age group over another, re-measuring the stimulation of the identified receptors for the target response in the context of the final consumer product to confirm the desired level of activity, and adjusting the concentration of the odorant composition based on interference or synergy with the consumer product mixture.

In other variations, the methods described herein are used to create an age-related odor or flavor profile for an odorant composition. The odorant compositions may be used in varying concentrations with a consumer product, for example, an age-restricted product. Here the method generally includes: 1) identifying one or more olfactory receptors that bind the odorant composition, where the odorant composition is differentially perceived by at least two age groups, and where identifying is performed by: a) exposing the one or more olfactory receptors to the odorant composition at a plurality of odorant concentrations, the one or more olfactory receptors being coupled to a reporter that produces a signal upon binding of the odorant composition to the one or more olfactory receptors, and b) detecting binding of the one or more olfactory receptors to the odorant composition at each odorant concentration by measuring the produced signal; 2) creating an age-related odor or flavor profile by determining the concentration range of the odorant composition resulting in a 10% to 90% maximal binding response to the one or more odorant receptors (i.e. EC₁₀ to EC₉₀ concentration range); and 3) designating for use the odorant composition in the product at the concentration that produces the desired effect in one of the at least two age groups.

The odorant composition may be included in a product at the concentration that produces the desired effect in a certain age group. The odorant composition may be included in a product at an EC₁₀ to an EC₉₀ concentration, an EC₂₀ to an EC₉₀ concentration, an EC₃₀ to an EC₉₀ concentration, an EC₄₀ to an EC₉₀ concentration, an EC₅₀ to an EC₉₀ concentration, an EC₆₀ to an EC₉₀ concentration, an EC₇₀ to an EC₉₀ concentration, an EC80 to an EC₉₀ concentration, or greater than an EC₉₀ concentration, depending on such factors as the application and receptor-odorant response levels, to produce a differential effect between persons of different ages (e.g., an older person and a younger person). For example, the concentration of the odorant composition included in a product may generate a negative effect that deters use of the product in children and adolescents but not adults. The concentrations of the odorant composition that may be included in a product to produce a differential effect, e.g., a negative effect, in one age group but not another, may the EC₁₀, EC₂₀, EC₃₀, EC₄₀, EC₅₀, EC₆₀, EC₇₀, EC₈₀, or EC90 concentration. Employing a concentration greater than EC₉₀ may be useful in some applications.

### ODORANT COMPOSITIONS

The odorant compositions described herein include one or more odorants. The compositions *per se* are not claimed, but are useful for practising the invention. The odorant compositions may have any suitable form, for example, the composition may be a liquid, solid, semi-solid, or gaseous form. In some variations, the odorant compositions are known to be differentially perceived by at least two age groups, but their corresponding olfactory receptors need to be identified. For example, compositions comprising menthol are generally known to be differentially perceived by children and adults, but their corresponding olfactory receptors require identification in order to determine effective concentrations for differential receptor activation. Other exemplary odorants known to be differentially perceived by certain age groups may include without limitation, mint, banana, onion, and rose.

In variations where odorant compositions are screened to determine whether they are differentially perceived by at least two age groups, the odorant that is differentially perceived may include without limitation, the entirety of, or component(s) of: caramel, honey, butterscotch, butter, soy sauce, chocolate, molasses, sulfur dioxide, ethanol, acetic acid, ethyl acetate, burnt match, cabbage, skunk, garlic, mercaptan, hydrogen sulfide, diesel, kerosene, plastic, tar, mold, cork, mushroom, dust, floral substances (e.g., geranium, violet, orange blossom, and lavender), fruit substances (e.g., grapefruit, lemon, blackberry, raspberry, strawberry, black currant, cherry, apricot, peach, apple, pineapple, melon, banana, strawberry jam, raisin, prune, fig, methyl anthranilate), herbs or vegetables (cut green grass, bell pepper, eucalyptus, green beans, asparagus, green olive, black olive, artichoke), hay, straw, tea, tobacco, walnut, hazelnut, almond, sherry, licorice, anise, black pepper, cloves, coffee, bacon, oak, cedar, and vanilla.

Screening to determine the ability of odorant compositions to activate (bind) olfactory receptors may include generating serial dilutions of the test odorant composition, testing each dilution against one or more receptors, and determining an EC₅₀ concentration. From this information, odorant compositions may be designed such that receptor signaling for the undiluted mixture is at the EC₁₀ to greater than EC₉₀ concentration for at least one of the activated receptors.

### OLFACTORY RECEPTORS

Any suitable olfactory receptor may be used in the methods described herein. Most olfactory receptors are G-protein coupled receptors that associate with a G-protein for signal transduction after the receptor is activated by an odorant. The discriminatory power of olfactory receptors is such that, together, they can bind thousands of volatile chemicals in millions of combinations, each with potentially different perceptions. It is known that the olfactory system uses a combinatorial receptor coding scheme to decipher the odor molecules. One olfactory receptor can recognize multiple odorants and one odorant can be recognized by multiple olfactory receptors. A slight structural change in the odorant or a change in the concentration of the odorant in the environment can also result in a change of how the odorant is perceived by an age group.

In some variations, the olfactory receptors are mammalian olfactory receptors such as human olfactory receptors. In other variations, the olfactory receptors are from other mammals, e.g., mice, rats, cats, dogs, cattle, horses, goats, pigs, and bears. In further variations, the olfactory receptors are hybrid olfactory receptors.

Exemplary human olfactory receptors, including single nucleotide polymorphism (SNP) variants, that may be used in the methods disclosed herein include those from the following 18 classified human families, and as further described below: OR1, OR2, OR3, OR4, OR5, OR6, OR7, OR8, OR9, OR10, OR11, OR12, OR13, OR14, OR51, OR52, OR55 and OR56.

Human family OR1 has 21 members: OR1A, OR1B, OR1C, OR1D, OR1E, OR1F, OR1G, OR1H, OR1I, OR1J, OR1K, OR1L, OR1M, OR1N, OR1P, OR1Q, OR1R, OR1S, OR1X, OR1AA, and OR1AB. Family OR2 has 41 members: OR2A, OR2B, OR2C, OR2D, OR2E, OR2F, OR2G, OR2H, OR21, OR2J, OR2K, OR2L, OR2M, OR2N, OR2Q, OR2R, OR2S, OR2T, OR2U, OR2V, OR2W, OR2X, OR2Y, OR2Z, OR2AD, OR2AE, OR2AF, OR2AG, OR2AH, OR2AI, OR2AJ, OR2AK, OR2AL, OR2AM, OR2AO, OR2AP, OR2AS, and OR2AT.

Human family OR3 has 3 members: OR3A, OR3B, and OR3D.

Human family OR4 has 21 members: OR4A, OR4B, OR4C, OR4D, OR4E, OR4F, OR4G, OR4H, OR4K, OR4L, OR4M, OR4N, OR4P, OR4Q, OR4R, OR4S, OR4T, OR4U, OR4V, OR4W, and OR4X.

Human family OR5 has 49 members: OR5A, OR5B, OR5C, OR5D, OR5E, OR5F, OR5G, OR5H, OR51, OR5J, OR5K, OR5L, OR5M, OR5P, OR5R, OR5S, OR5T, OR5V, OR5W, OR5AC, OR5AH, OR5AK, OR5AL, OR5AM, OR5AN, ORSAO, OR5AP, OR5AQ, OR5AR, OR5AS, OR5AU, OR5W, OR5X, OR5Y, OR5Z, OR5BA, OR5BB, OR5BC, OR5BD, OR5BE, OR5BH, OR5BJ, OR5BK, OR5BL, OR5BM, OR5BN, OR5BP, OR5BQ, OR5BR, OR5BS, and OR5BT.

Human family OR6 has 21 members: OR6A, OR6B, OR6C, OR6D, OR6E, OR6F, OR6J, OR6K, OR6L, OR6M, OR6N, OR6P, OR6Q, OR6R, OR6S, OR6T, OR6U, OR6V, OR6W, OR6X, and ORGY.

Human family OR7 has 9 members: OR7A, OR7C, OR7D, OR7E, OR7G, OR7H, OR7K, OR7L, and OR7M.

Human family OR8 has 18 members: OR8A, OR8B, OR8C, OR8D, OR8F, OR8G, OR8H, OR81, OR8J, OR8K, OR8L, OR8Q, OR8R, ORBS, OR8T, OR8U, OR8V, and OR8X.

Human family OR9 has 12 members: OR9A, OR9G, OR9H, OR9J, OR9K, OR9L, OR9M, OR9N, OR9P, OR9Q, OR9R, and OR9S.

Human family OR10 has 29 members: OR10A, OR10B, OR10C, OR10D, OR10G, OR10H, OR10J, OR10K, OR10N, OR10P, OR10Q, OR10R, OR10S, OR10T, OR10U, OR10V, OR10W, OR10X, OR10Y, OR10Z, OR10AA, OR10AB, OR10AC, OR10AD, OR10AE, OR10AF, OR10AG, OR10AH, and OR10AK.

Human family OR11 has 11 members: OR11A, OR11G, OR11H, OR11I, OR11J, OR11K, OR11L, OR11M, OR11N, OR11P, OR11Q.

Human family OR12 has 1 member: OR12D.

Human family OR13 has 11 members: OR13A, OR13C, OR13D, OR13E, OR13F, OR13G, OR13H, OR131, OR13J, OR13K, and OR13Z.

Human family OR14 has 6 members: OR14A, OR14C, OR141, OR14J, OR14K, and OR14L.

Human family OR51 has 21 members: OR51A, OR51B, OR51C, OR51D, OR51E, OR51F, OR51G, OR51H, OR511, OR51J, OR51K, OR51L, OR51M, OR51N, OR51P, OR51Q, OR51R, OR51S, OR51T, OR51V, and OR51AB.

Human family OR52 has 22 members: OR52A, OR52B, OR52D, OR52E, OR52H, OR521, OR52J, OR52K, OR52L, OR52M, OR52N, OR52P, OR52Q, OR52R, OR52S, OR52T, OR52U, OR52V, OR52W, OR52X, OR52Y, and OR52Z.

Human family OR55 has 1 member: OR55B.

Human family OR56 has 2 members: OR56A and OR56B.

Ectopic olfactory receptors may also be used in the methods described herein. These receptors are generally the same in genetic sequence as olfactory receptors in the nose but expressed in organs, tissues, or cells that do not play a role in olfaction. For example, ectopic olfactory receptors have been found in many non-olfaction tissues and cells including, skin, brain, breast, colon, erythroid cells, eye, heart, kidney, liver, lung, ovary, prostate, spleen, testis, white blood cells, lymph nodes, adrenal tissue, adipose tissue, and neural tissue. The ectopic olfactory receptors may also be olfactory receptors from another mammal.

Exemplary ectopic olfactory receptors found in adipose include, for example, OR51E2, OR2W3, OR51E1, OR2A1/42, OR2A4/7, OR52N4, OR13A1, 047D2, OR10J1OR1L8, OR2B6, OR4D6, TASIR3, TAS2R10, TAS2R13, TAS2R14, TAS2R19, TAS2R20, TAS2R31, TAS2R40, TAS2R42, TAS2R5, VN1R1, and VN1R2.

Ectopic olfactory receptors found in adrenal tissue include, for example, OR51E2, ORW3, OR51E1, OR2A1/42, OR2A4/7, OR52N4, OR13A1, OR5K2, OR3A2, OR2H2, OR7C1, OR2L13, ORIL8, OR2T8, OR10AD1, OR52B6, OR1E1, OR13J1, OR2C1, OR52D1, OR10A2, OR2B6, OR8G5, OR1F12, OR4D6, TAS1R1, TASIR3, TAS2R10, TAS2R13, TAS2R14, TAS2R19, TAS2R20, TAS2R3, TAS2R30, TAS2R31, TAS2R4, TAS2R42, TAS2R5, TAS2R50, TAS2R9, and VN1R1.

Ectopic olfactory receptors found in central nervous system organs and tissues include, for example, OR51E2, OR2W3, OR4N4, OR51E1, OR52N4, OR13A1, OR5K2, OR7D2, OR3A2, OR2V1, OR2H2, OR7C1, OR2L13, ORIL8, OR2T8, OR10AD1, OR3A3, OR2K2, OR13J1, OR2C1, OR7A5, OR10A2, OR1F12, TAAR3, TAAR5, TAAR6. TASIRI, TAS1R3, TAS2R1, TAS2R10, TAS2R13, TAS2R14, TAS2R19, TAS2R20, TAS2R3, TAS2R30, TAS2R31, TAS2R39, TAS2R4, TAS2R40, TAS2R42, TAS2R46, TAS2R5, TAS2R50, TAS2R7, TAS2R8, TAS2R9, VN1R1, VNIR2, and VN1R5.

Ectopic olfactory receptors found in dopaminergic neurons include, for example, OR51E1, OR51E2, and OR2J3. Ectopic Olfactory Receptors found in breast include, for example, OR51E2, OR51E1, OR2A1/42, OR2A4/7, OR52N4, OR5K2, OR3A2, OR2T8, OR10AD1, OR3A3, OR2K2, OR1E1, OR2C1, OR2C3, OR8D1, OR7A5, OR10A2, TAS1R1, TAS1R3, TAS2R10, TAS2R13, TAS2R14, TAS2R19, TAS2R20, TAS2R31, TAS2R4, TAS2R5, and VN1R1.

Ectopic olfactory receptors found in colon include, for example, OR51E2, OR2W3, OR51E1, OR2A1/42, OR2A4/7, OR5K2, OR7D2, OR7C1, OR2L13, OR7A5, OR51B5, TAS1R1, TAS1R3, TAS2R14, TAS2R20, TAS2R4, TAS2R43, TAS2R5, and VN1R1.

Ectopic olfactory receptors found in heart tissue include, for example, OR51E2, OR51E1, OR52N4, OR13A1, OR2H2, OR10AD1, OR3A3, OR52B6, OR2K2, OR8G5, OR4D6, TAS1R1, TASIR3, TAS2R10, TAS2R13, TAS2R14, TAS2R19, TAS2R20, TAS2R3, TAS2R30, TAS2R31, TAS2R4, TAS2R43, TAS2R46, TAS2R5, TAS2R50, TAS2R7, and VN1R1.

Ectopic olfactory receptors found in kidney include, for example, OR51E2, OR51E1, OR2A1/42, OR2A4/7, OR5K2, ORIL8, OR10A2, OR1F12, TAS1R1, TAS1R3, TAS2R1, TAS2R10, TAS2R14, TAS2R19, TAS2R20, TAS2R3, TAS2R30, TAS2R31, TAS2R4, TAS2R42, TAS2R43, TAS2R5, TAS2R50, and VN1R1.

Ectopic olfactory receptors found in liver include, for example, OR2W3, OR51E1, OR2A1/42, OR2A4/7, OR7D2, OR1L8, OR2T8. TASIR3, TAS2R14, TAS2R14, TAS2R20, TAS2R30, TAS2R30, TAS2R40, TAS2R5, VN1R1, and VN1R2.

Ectopic olfactory receptors found in lymph node tissue include, for example, OR51E2, OR51E1, OR2A1/42, OR52N4, OR13A1, OR5K2, OR3A2, OR2H2, OR3A3, OR2B6, TAS1R3, TAS2R14, TAS2R19, TAS2R20, TAS2R31, TAS2R4, TAS2R40, TAS2R43, TAS2R5, and VN1R1.

Ectopic Olfactory Receptors found in ovarian tissue include, for example, OR51E2, OR2W3, OR4N4, OR51E1, OR2A1/42, OR2A4/7, OR52N4, OR5K2, OR3A2, OR2V1, OR2H2, OR2L13, OR1L8, OR10AD1, OR3A3, OR52B6, OR13J1, OR2C1, OR52D1, OR5lB5, OR1F12, TAS1R1, TASIR3, TAS2R1, TAS2R10, TAS2R13, TAS2R14, TAS2R19, TAS2R20, TAS2R3, TAS2R31, TAS2R4, TAS2R42, TAS2R43, TAS2R5, TAS2R50, TAS2R60, TAS2R7, VN1R1, and VN1R2.

Ectopic olfactory receptors found in prostate include, for example, OR51E2, OR2W3, OR51E1, OR2A1/42, OR2A4/7, OR52N4, OR13A1, OR5K2, OR2H2, OR7C1, OR1E1, OR13J1, OR51B5, TAS1R3, TAS2R14, TAS2R19, TAS2R20, TAS2R43, TAS2R46, TAS2R5, and VN1R1.

Ectopic olfactory receptors found in skin include, for example, OR2AT4 and OR51E2.

Ectopic olfactory receptors found in testis include, for example, OR4N4, OR6F1, OR2H1, OR51E2, OR2W3, OR4N4, OR51E1, OR2A1/42, OR2A4/7, OR52N4, OR7D2, OR3A2, OR2V1, OR2H2, OR7C1, OR10J1, ORIL8, OR1C1, OR2H1, OR10AD1, OR3A3, OR13C3, OR2K2, OR1E1, OR2C1, OR2K2, OR1E1, OR2C1, OR2C3, OR8D1, OR52D1, OR7A5, OR10A2, OR2B6, OR7E24, OR6Fl, OR8G5, OR51B5, OR1F12, TAS1R1, TASIR3, TAS2R1, TAS2R14, TAS2R19, TAS2R20, TAS2R3, TAS2R31, TAS2R4, TAS2R43, TAS2R5, TAS2R50 TAS2R60, VN1R1, VNIR2, VN1R3, and VN1R4.

Ectopic olfactory receptors found in white blood cells include, for example, OR2W3, OR2A4/7, OR52N4, OR7D2, OR2L13, OR3A3, OR2C1, OR2C3, OR2B6, TAS1R3, TAS2R14, TAS2R20, TAS2R40, and TAS2R60.

In yet further variations, the olfactory receptors are engineered receptors. For example, amino acids from the N-terminal region of one olfactory receptor may be fused to the N-terminal region of a second, different olfactory receptor. In some variations, the N-terminal amino acids are from amino acid positions 1-61 of the donor olfactory receptor. In other variations, the N-terminal amino acids are from amino acid positions 1-55 of the donor olfactory receptor. In one variation, the N-terminal amino acids are from amino acid positions 1-20 or the amino acids up to the first transmembrane domain, or amino acid positions 1-40 which includes the consensus sequence of the first transmembrane domain. In another variation, the N-terminal amino acids are fused to the acceptor olfactory receptor at its N-terminal region of amino acid positions 1-61. Amino acids from the C-terminus of a donor polypeptide may also be fused to the C-terminal end of the acceptor olfactory receptor. In some variations, 1-50 amino acids from the C-terminus of the acceptor olfactory receptor are replaced by amino acids from a donor polypeptide. In other variations, 1-55 amino acids from the C-terminus of the acceptor olfactory receptor are replaced by amino acids from a donor polypeptide. The donor polypeptide may be another olfactory receptor. Various tags may also be included in the engineered olfactory receptors. For example, the olfactory receptors may be fused at their N- or C- terminal end with a FLAG, HIS, RHO, or LUCY tag to assist in certain purification steps and biochemical characterization processes.

In some variations, the one or more olfactory receptors are OR51E1, OR8K3, OR2W1, OR2J2, OR11H7P, OR5P3, or OR1G1. These receptors may be useful in binding or identifying odorant compositions including menthol or mint. Olfactory receptors OR52D1 and OR1G1 may be useful in binding or identifying compositions including isoamyl acetate or banana.

### AGE-RELATED ODOR OR FLAVOR PROFILES

The odorant compositions and olfactory receptors mentioned above may be used to create an age-related odor or flavor profile for the odorant compositions. The profiles may then help determine the effective concentration range of an odorant composition to include in a consumer product to obtain a desired effect in certain age groups. The concentration may be selected to generate a positive effect, where the odorant is generally considered appealing by an age group, or selected to generate a negative effect, where the odorant is generally considered unappealing to an age group. In one variation, the odorant composition is differentially perceived by at least two age groups based on the concentration differences.

Methods for determining the concentration of an odorant composition for use with a product may include: 1) identifying one or more olfactory receptors that bind the odorant composition, where the odorant composition is differentially perceived by at least two age groups , and wherein identifying is performed by: a) exposing the one or more olfactory receptors to the odorant composition at a plurality of odorant concentrations, the one or more olfactory receptors being coupled to a reporter that produces a signal upon binding of the odorant composition to the one or more olfactory receptors; and b) detecting binding of the one or more olfactory receptors to the odorant composition at each odorant concentration by measuring the produced signal; 2) creating an age-related odor or flavor profile by determining concentration range of the odorant composition resulting in a 10% to 90% maximal binding response to the one or more odorant receptors (i.e. EC₁₀ to EC₉₀ concentration range); and 3) designating for use the odorant composition in the product at the concentration that produces the desired effect in one of the at least two age groups.

The odorant composition may be included in a product at the concentration that produces the desired effect in a certain age group, as previously stated. The effect may be different between at least two age groups based on the concentration employed. For example, the odorant composition may be included in a product at a concentration to produce a negative effect that deters use of the product in children and adolescents but not adults. Age-restricted products may include alcohol, cannabis, nicotine, or tobacco. For example, age-restricted products containing nicotine include without limitation, electronic cigarettes and other vaping devices, and lit-end cigarettes.

In one variation, the odorant composition includes menthol at the EC₈₀ to greater than EC₉₀ concentration to deter use of an age-restricted product by children and adolescents. Menthol is one odorant that is generally known as being differentially perceived between at least two age groups (adults and children). Adults require approximately two times higher menthol concentrations in order to perceive the same level of intensity as children. This effect may be due to downregulation of the receptor or other interference along the perception neurological pathway. This observation of declining olfaction capability is similar to the phenomenon of adults losing sensitivity to certain auditory frequencies as they age. Creation of an age-related odor or flavor profile for an odorant composition including menthol is further detailed in Example 1.

Odorant compositions may also be screened against menthol-sensitive receptors (e.g., OR51E1) to determine whether they may be differentially perceived by certain age groups. For example, a series of odorants may be screened against OR51E1 and other menthol-related ORs to identify odorants that more specifically target these receptors within a certain concentration range. The best identified odorant is then added at multiple concentrations to a target consumer product, and the activation curve of the odorant concentration versus receptor stimulation is compared with and without the consumer product to assess the effect of other components of the consumer product on the sensitivity of the receptor to the odorant. An optimal concentration of the odorant may then be chosen that provides high stimulation of the menthol receptor(s) sufficient to be perceived strongly (i.e., negatively) by younger consumers but will be perceived less strongly by adults with reduced sensory perception via the target receptors.

In some variations, the differentially perceived odorant compositions may be used in certain environments to attract or increase their appeal to certain age groups. For example, these compositions may be used in the home to provide desired responses (e.g., relaxation) for either adults or children in the home.

When employed in the home environment, differentially perceived odorant compositions may be delivered by placing the compositions in a material of a product (e.g., a plastic, leather, cloth, wood, or wax part, etc.) from which the odor is slowly released over time. In some variations, the odor is released from the material when it is subjected to a different set of conditions. For example, the odor could be placed in a material where it is released upon heating of the material (e.g., similar to Glad plug-ins). Other changes in conditions besides temperature may also be used to release the odor, including, for example, a solvent, or a change in pH, airflow, or light.

When creating an age-related odor or flavor profile, one or more olfactory receptors are exposed to an odorant composition at a plurality of odorant concentrations, and binding of the one or more olfactory receptors is detected based on measuring a signal produced by a reporter that is coupled to the one or more receptors. A reporter or reporter molecule may be a moiety capable of being detected indirectly or directly. Reporters include, without limitation, a chromophore, a fluorophore, a fluorescent protein, a luminescent protein, a receptor, a hapten, an enzyme, and a radioisotope. The signals produced by the reporters may be detected or measured using various techniques, for example, via a standard plate reader, binary colorimetric characterization, etc.

In some variations, the reporter or reporters are one or more of a fluorescent reporter, a bioluminescent reporter, an enzyme, and an ion channel. Examples of fluorescent reporters include without limitation, green fluorescent protein from *Aequorea Victoria* or *Renilla reniformis,* and active variants thereof (e.g., blue fluorescent protein, yellow fluorescent protein, cyan fluorescent protein, etc.); fluorescent proteins from Hydroid jellyfishes, Copepod, Ctenophora, Anthrozoas, and Entacmaea quadricolor, and active variants thereof; and phycobiliproteins and active variants thereof. Other fluorescent reporters include, for example, small molecules such as CPSD (Disodium 3-(4-methoxyspiro {1,2-dioxetane-3,2'-(5'-chloro)tricyclo [3.3.1.1^{3.7}]decan}-4-yl)phenyl phosphate, ThermoFisher Catalog # T2141). Bioluminescent reporters include, for example, aequorin (and other Ca⁻² regulated photoproteins), luciferase based on luciferin substrate, luciferase based on Coelenterazine substrate (e.g., *Renilla. Gaussia,* and *Metridina*), and luciferase from *Cypridina*, and active variants thereof. In some variations, the bioluminescent reporter may be the North American firefly luciferase, Japanese firefly luciferase, Italian firefly luciferase, East European firefly luciferase, Pennsylvania firefly luciferase, Click beetle luciferase, railroad worm luciferase, *Renilla* luciferase, *Gaussia* luciferase, *Cypridine* luciferase, *Metrida* luciferase, or OLuc, and red firefly luciferase, all of which are commercially available from ThermoFisher Scientific and/or Promega.

Exemplary enzyme reporters that may be used include β-galactosidase, chloramphenicol acetyltransferase, horseradish peroxidase, alkaline phosphatase, acetylcholinesterase, and catalase. Ion channel reporters that may be employed, include, for example, cAMP activated cation channels. The reporter or reporters may also include a Positron Emission Tomography (PET) reporter, a Single Photon Emission Computed Tomography (SPECT) reporter, a photoacoustic reporter, an X-ray reporter, or an ultrasound reporter.

The age-related odor or flavor profiles may be created using assays in which one or more olfactory receptors are part of a live cell assay that uses yeast or mammalian cells. Alternatively, the olfactory receptors may be provided in an assay that uses membrane fractions of a host cell. An exemplary method for making yeast membrane fractions is described in Example 2. In some instances, the one or more olfactory receptors may be provided in liposomes (proteoliposomes), which may be made as described in Example 3.

Host cells may be prokaryotic or eukaryotic cells into which the vectors of the invention may be introduced, expressed and/or propagated. A microbial host cell is a cell of a prokaryotic or eukaryotic microorganism, including bacteria, yeasts, microscopic fungi and microscopic phases in the life-cycle of fungi and slime molds. Typical prokaryotic host cells include various strains of *E. coli.* Typical eukaryotic host cells are yeast or filamentous fungi, or mammalian cells, such as Chinese hamster cells, murine NIH 3T3 fibroblasts, human kidney cells, or rodent myeloma or hybridoma cells.

Exemplary eukaryotic cells that may be used as the host cell include without limitation, a fungal cell, an animal cell, a plant cell, and an algae cell. In some variations, the eukaryotic cells are fungi cells, including, but not limited to, fungi of the genera *Aspergillus, Trichoderma, Saccharomyces, Chirysosporium, Klyuveromyces, Candida, Pichia, Debaromyces, Hansenula, Yarrowia, Zygosaccharomyces, Schizosaccharomyees, Penicillium,* or *Rhizopus.* In other variations, the fungi cells are from *Saccharomyces cerevisiae, Pichia pastoris, Aspergillus niger, Aspergillus ormae, Chrysogorium lucknowense,* or *Trichoderma reesei.*

In further variations, the host cells are animal cells. The animal cells may be mammalian cells, such as that of bovine, canine, feline, hamster, mouse, porcine, rabbit, rat, or sheep origin. In some variations, the mammalian cells are cells of primates, including but not limited to, monkeys, chimpanzees, gorillas, and humans. More specifically, animal cells may include, fibroblasts, epithelial cells (e.g., renal, mammary, prostate, lung), keratinocytes, hepatocytes, adipocytes, endothelial cells, hematopoietic cells. In some variations, the animal cells are adult cells (e g., terminally differentiated, dividing or non-dividing) or stem cells. In other variations, mammalian cell lines are used as host cells. The cell lines may be derived from Chinese hamster cells, Human kidney cells, Monkey kidney cells, Human cervical cancer cells, or Mouse myeloma cells.

The eukaryotic cells may also be plant cells. In some variations, the plant cells are cells of monocotyledonous or dicotyledonous plants, including, but not limited to, alfalfa, almonds, asparagus, avocado, banana, barley, bean, blackberry, brassicas, broccoli, cabbage, canola, carrot, cauliflower, celery, cherry, chicory, citrus, coffee, cotton, cucumber, eucalyptus, hemp, lettuce, lentil, maize, mango, melon, oat, papaya, pea, peanut, pineapple, plum, potato (including sweet potatoes), pumpkin, radish, rapeseed, raspberry, rice, rye, sorghum, soybean, spinach, strawberry, sugar beet, sugarcane, sunflower, tobacco, tomato, turnip, wheat, zucchini, and other fruiting vegetables (e.g., tomatoes, pepper, chili, eggplant, cucumber, squash etc.), other bulb vegetables (e.g., garlic, onion, leek etc.), other pome fruit (e.g., apples, pears etc.), other stone fruit (e.g., peach, nectarine, apricot, pears, plums etc.), Arabidopsis, woody plants such as coniferous and deciduous trees, an ornamental plant, a perennial grass, a forage crop, flowers, other vegetables, other fruits, other agricultural crops, herbs, grass, or perennial plant parts (e.g., bulbs, tubers; roots; crowns; stems; stolons; tillers; shoots; cuttings, including un-rooted cuttings, rooted cuttings, and callus cuttings or callus-generated plantlets; apical meristems, etc.).

In some embodiments, the eukaryotic cells are algal, including but not limited to algae of the genera *Chlorella, Chlamydomonas, Scenedesmus, Isochrysis, Dunaliella, Tetraselmis, Nannochloropsis,* or *Prototheca.*

### EXAMPLES

The following examples are for illustrative purposes only.

### EXAMPLE 1: AGE-RELATED FLAVOR PROFILE CREATION FOR MENTHOL

Menthol is well established in the literature as being perceived less intensely by humans as they age, in both flavor and odor. As odor and flavor perception of menthol are controlled by binding of the compound to a subset of human olfactory receptors (in the presence or absence of other odorants), one or more menthol-sensitive olfactory receptors are likely reduced in their signaling to the brain in older persons relative to younger persons. This reduction in perception is currently poorly understood and may be due to a number of factors. For example, it may reflect a downregulation of expression of the receptor genes, toxicity or necrosis of specific neurons expressing the receptors, or changes in the olfactory bulb signal processing leading to a reduction in receptor signaling to the brain.

When creating an age-related flavor profile for menthol, identification of the olfactory receptors that respond to menthol may be accomplished by transfecting human olfactory receptor cDNA sequences in HEK293T co-expressing accessory factors for odorant receptor expression (see, e.g., Saito, H., et al., Cell 119:679-691 (2004)). Transfected cells may then be exposed to serial dilutions of menthol from 1 nM to 1 mM in growth medium, and receptor signaling may be measured by cAMP production in the cell using a commercial assay.

Next, positive olfactory receptors may be identified by increased signaling when exposed to at least one menthol concentration compared to control cells that have not been transfected. In each case, an EC₅₀ is defined for the receptor versus menthol. Olfactory receptors OR1G1, 8K3, 2W1, 2J2, 11H7P and 51E1 have been identified by this method as menthol-sensitive olfactory receptors.

When developing age-discriminating odorant (flavor) compositions, the odorant compositions may be screened for their ability to activate menthol-sensitive receptors (e.g., OR1G1, 8K3, 2W1, 2J2, 11H7P, and 51E1). In each case, serial dilutions of the test odorant composition may be tested against each menthol-sensitive receptor and an EC₅₀ determined. From this information, odorant mixtures may be designed such that receptor signaling for the undiluted mixture is at between EC₈₀ and EC₉₀ for at least one of the menthol sensitive receptors. These mixtures may also comprise other odorants that are more pleasant, representing the desired flavor perception of the end product.

Success of the design may then be assessed by testing the designed odorant compositions against human sensory panels representing different age groups. For those odorant compositions at the EC₈₀ to EC₉₀ concentration for an age discriminating receptor such as menthol receptors, younger persons will have the highest sensitivity to detection and will perceive a strong signal via the target receptors, which may result in a negative response to the otherwise pleasant odorant. As the sensitivity of older persons is reduced, the EC₅₀ of the receptor is shifted to higher concentrations and the apparent signal from the designed mixture may be less intense for older individuals and therefore not be perceived as unpleasant.

### EXAMPLE 2: PREPARATION OF MEMBRANE FRACTIONS FROM YEAST CELLS

Membrane fractions from yeast cells may be prepared by lysis with glass beads in a blender, as outlined below.
1. Start with yeast cells that have been induced in galactose to express an olfactory receptor.
   a) Record OD₆₆₀ and original culture volume.
2. Spin down pellet and discard supernatant in 50mL centrifuge tube until all cells of the same type are in that tube.
3. Wash cells three times in PBS (PBS salts in IL of ddH₂O) by spinning down at 3000 rpm (1250 rcf) for 4 minutes and discard supernatant.
4. Resuspend cell pellet in 10mL of cell lysis buffer (8.5mL PBS, 250µL Protease Inhibitor Cocktail IV, 500µL PMSF).
5. Lyse cells with glass beads.
   a) Add cells in cell lysis buffer to blender well.
   b) Fill well halfway up with glass beads. Fill up to top of metal nut with lysis buffer.
   c) Cap well and put in the metal inner chamber with ice filling around it.
   d) Screw well and inner chamber into plastic outer chamber and fill with ice.
   e) Place on blender and run 7 times alternating between 30 seconds on low and 3 minutes off.
6. Transfer lysate from well to 15mL centrifuge tube and rinse beads with 1mL of cell lysis buffer and add that to lysate.
7. Wash out glass beads into an Erlenmeyer flask.
8. Spin lysate down at 4830 rpm (3000 ref) for 10 minutes at 4°C and collect supernatant.
9. Aliquot supernatant into ultracentrifuge topless 3mL tubes and weigh.
10. Spin tubes for 1 hour at 43,000 rpm on ultracentrifuge at 4°C to obtain pellet with membrane fractions.

When membrane fractions are to be used for further experiments, re-suspend pellet with 990µL of water.

### EXAMPLE 3: PREPARATION OF PROTEOLIPOSOMES

Liposomes including olfactory receptors may be formed, as outlined below.
1. Yeast lipids (e.g., from Avanti^{®} total yeast extract) are first resuspended in yeast lysis buffer in a test tube. The test tube with the lipid solution is then placed in a 50 mL centrifuge tube holding 45 mL of water being agitated by a sonicator. As liposomes are formed, the solution becomes clearer.
2. Yeast membrane fractions made as described in Example 2 are then added to the liposomes.
3. Add between about 25% to about 90% yeast membrane with olfactory receptors to the yeast lipids to form a suspension.
4. Extrude the lipid suspension using a 5000 nm then 1000 nm pore size extruder. The extruder should be maintained between 55°C to 60°C. Repeat extrusion of the lipid suspension at least four times.
5. Place the lipid suspension in to a clean vial and store at 4°C until used for further experimentation.

## Claims

1. An *in vitro* method for determining the concentration of an odorant composition for use with a product comprising:
identifying one or more olfactory receptors that bind the odorant composition, wherein the odorant composition is differentially perceived by at least two age groups, and wherein identifying is performed by:
exposing the one or more olfactory receptors to the odorant composition at a plurality of odorant concentrations, the one or more olfactory receptors being coupled to a reporter that produces a signal upon binding of the odorant composition to the one or more olfactory receptors; and
detecting binding of the one or more olfactory receptors to the odorant composition at each odorant concentration by measuring the produced signal;
creating an age-related odor or flavor profile by determining the concentration range of the odorant composition resulting in a 10% to 90% maximal binding response to the one or more odorant receptors (i.e. EC₁₀ to EC₉₀ concentration range) and relating concentrations in the range to a differential effect for the different age groups; and
designating for use the odorant composition in the product at the concentration that produces the desired effect in one of the at least two age groups.

2. The method of claim 1, wherein the odorant composition at the EC₉₀ concentration is designated for use in the product by one of the at least two age groups.

3. The method of claim 1, wherein the at least two age groups comprise children, adolescents or adults.

4. The method of claim 1, wherein the odorant composition comprises one or more odorants.

5. The method of claim 4, wherein the one or more odorants comprises menthol, mint, banana, onion, or rose.

6. The method of claim 1, wherein the odorant composition comprises menthol.

7. The method of claim 1, wherein the one or more olfactory receptors is OR51E1, OR8K3, OR2W1, OR2J2, OR11H7P, OR5P3, or OR1G1.

8. The method of claim 1, wherein the one or more olfactory receptors is OR52D1 or OR1G1.

9. The method of claim 1 or 6, wherein the product is an age-restricted product.

10. The method of claim 9, wherein the age-restricted product comprises alcohol, cannabis, nicotine, or tobacco.

11. The method of claim 1, wherein the one or more olfactory receptors are part of a live cell assay.

12. The method of claim 1, wherein the one or more olfactory receptors are provided in a membrane fraction of a host cell.

13. The method of claim 1, wherein the one or more olfactory receptors are provided in liposomes.

14. A method according to claim 9 when dependent on claim 6, wherein the EC₈₀ concentration to the EC₉₀ concentration is designated for use to deter the age-restricted product by children and/or adolescents.

## Patentansprüche

1. Ein in vitro-Verfahren zur Bestimmung der Konzentration einer Geruchsstoffzusammensetzung zur Verwendung mit einem Produkt, umfassend:
Identifizieren eines oder mehrerer olfaktorischer Rezeptoren, die die Geruchsstoffzusammensetzung binden, wobei die Geruchsstoffzusammensetzung von mindestens zwei Altersgruppen unterschiedlich wahrgenommen wird und wobei das Identifizieren durchgeführt wird durch:
Aussetzen des einen oder der mehreren olfaktorischen Rezeptoren der Geruchsstoffzusammensetzung in einer Mehrzahl von Geruchsstoffkonzentrationen, wobei der eine oder die mehreren olfaktorischen Rezeptoren mit einem Reporter gekoppelt sind, der bei Bindung der Geruchsstoffzusammensetzung an den einen oder die mehreren olfaktorischen Rezeptoren ein Signal erzeugt; und
Nachweis der Bindung des einen oder der mehreren olfaktorischen Rezeptoren an die Geruchsstoffzusammensetzung bei jeder Geruchsstoffkonzentration durch Messen des erzeugten Signals;
Erstellen eines altersbezogenen Geruchs- oder Geschmacksprofils durch Bestimmen des Konzentrationsbereichs der Geruchsstoffzusammensetzung, der zu einer maximalen Bindungsreaktion von 10% bis 90% an den einen oder die mehreren Geruchsstoffrezeptoren führt (d.h. Konzentrationsbereich von EC₁₀ bis EC₉₀) und Zuordnen von Konzentrationen in dem Bereich zu einer unterschiedlichen Wirkung für die verschiedenen Altersgruppen; und
Bestimmung der Geruchsstoffzusammensetzung zur Verwendung in dem Produkt bei der Konzentration, die die gewünschte Wirkung in einer der mindestens zwei Altersgruppen erzeugt.

2. Das Verfahren nach Anspruch 1, wobei die Geruchsstoffzusammensetzung bei der EC₉₀-Konzentration zur Verwendung in dem Produkt durch eine der mindestens zwei Altersgruppen bestimmt ist.

3. Das Verfahren nach Anspruch 1, wobei die mindestens zwei Altersgruppen Kinder, Jugendliche oder Erwachsene umfassen.

4. Das Verfahren nach Anspruch 1, wobei die Geruchsstoffzusammensetzung einen oder mehrere Geruchsstoffe umfasst.

5. Das Verfahren nach Anspruch 4, wobei der eine oder die mehreren Geruchsstoffe Menthol, Minze, Banane, Zwiebel oder Rose umfassen.

6. Das Verfahren nach Anspruch 1, wobei die Geruchsstoffzusammensetzung Menthol umfasst.

7. Das Verfahren nach Anspruch 1, wobei der eine oder die mehreren olfaktorischen Rezeptoren OR51E1, OR8K3, OR2W1, OR2J2, OR11H7P, OR5P3 oder OR1G1 sind.

8. Das Verfahren nach Anspruch 1, wobei der eine oder die mehreren olfaktorischen Rezeptoren OR52D1 oder OR1G1 sind.

9. Das Verfahren nach Anspruch 1 oder 6, wobei das Produkt ein Produkt mit Altersbeschränkung ist.

10. Das Verfahren nach Anspruch 9, wobei das Produkt mit Altersbeschränkung Alkohol, Cannabis, Nikotin oder Tabak umfasst.

11. Das Verfahren nach Anspruch 1, wobei der eine oder die mehreren olfaktorischen Rezeptoren Teil eines Lebendzell-Assays sind.

12. Das Verfahren nach Anspruch 1, wobei der eine oder die mehreren olfaktorischen Rezeptoren in einer Membranfraktion einer Wirtszelle bereitgestellt sind.

13. Das Verfahren nach Anspruch 1, wobei der eine oder die mehreren olfaktorischen Rezeptoren in Liposomen bereitgestellt sind.

14. Ein Verfahren nach Anspruch 9, abhängig von Anspruch 6, wobei die EC₈₀-Konzentration zu der EC₉₀-Konzentration zur Verwendung zum Abschrecken von Kindern und/oder Jugendlichen vor dem Produkt mit Altersbeschränkung bestimmt ist.

## Revendications

1. Méthode *in vitro* de détermination de la concentration d'une composition odorante pour l'utilisation avec un produit, comprenant :
l'identification d'un ou de plusieurs récepteurs olfactifs qui lient la composition odorante, où la composition odorante est perçue de manière différente par au moins deux groupes d'âges, et où l'identification est réalisée par :
l'exposition des un ou plusieurs récepteurs olfactifs à la composition odorante à une pluralité de concentrations odorantes, les un ou plusieurs récepteurs olfactifs étant couplés à un rapporteur qui produit un signal lors de la liaison de la composition odorante aux un ou plusieurs récepteurs olfactifs ; et
la détection de la liaison des un ou plusieurs récepteurs olfactifs à la composition odorante à chaque concentration par la mesure du signal produit ;
la création d'un profil d'odeur ou d'arôme lié à l'âge par détermination de la plage de concentration de la composition odorante donnant une réponse de liaison maximale de 10 % à 90 % aux un ou plusieurs récepteurs olfactifs (à savoir la plage de concentrations CE₁₀ à CE₉₀) et concernant des concentrations dans la plage à un effet différentiel pour les groupes d'âges différents ; et
la conception pour l'utilisation de la composition odorante dans le produit à la concentration qui produit l'effet désiré dans l'un des au moins deux groupes d'âges.

2. Méthode selon la revendication 1, dans laquelle la composition odorante à la concentration CE₉₀ est conçue pour l'utilisation dans le produit par l'un des au moins deux groupes d'âges.

3. Méthode selon la revendication 1, dans laquelle les au moins deux groupes d'âges comprennent des enfants, des adolescents ou des adultes.

4. Méthode selon la revendication 1, dans laquelle la composition odorante comprend un ou plusieurs agents odorants.

5. Méthode selon la revendication 4, dans laquelle les un ou plusieurs agents odorants comprennent le menthol, la menthe, la banane, l'oignon ou la rose.

6. Méthode selon la revendication 1, dans laquelle la composition odorante comprend du menthol.

7. Méthode selon la revendication 1, dans laquelle les un ou plusieurs récepteurs olfactifs sont OR51E1, OR8K3, OR2W1, OR2J2, OR11H7P, OR5P3, ou OR1G1.

8. Méthode selon la revendication 1, dans laquelle les un ou plusieurs récepteurs olfactifs sont OR52D1 ou OR1G1.

9. Méthode selon la revendication 1 ou 6, dans laquelle le produit est un produit interdit aux mineurs.

10. Méthode selon la revendication 9, dans laquelle le produit interdit aux mineurs comprend l'alcool, le cannabis, la nicotine, ou le tabac.

11. Méthode selon la revendication 1, dans laquelle les un ou plusieurs récepteurs olfactifs font partie d'un assay de cellules vivantes.

12. Méthode selon la revendication 1, dans laquelle les un ou plusieurs récepteurs olfactifs sont fournis dans une fraction membranaire d'une cellule hôte.

13. Méthode selon la revendication 1, dans laquelle les un ou plusieurs récepteurs olfactifs sont fournis dans des liposomes.

14. Méthode selon la revendication 9, lorsqu'elle est dépendante de la revendication 6, dans laquelle la concentration CE₈₀ sur la concentration CE₉₀ est conçue pour l'utilisation afin de dissuader la prise du produit interdit aux mineurs par les enfants et/ou les adolescents.
